# EUROPEAN PATENT SPECIFICATION

(11) **EP 3 064 941 B1**
(45) Date of publication and mention of the grant of the patent: **06.12.2017**
(21) Application number: 16158013.9
(22) Date of filing: 01.03.2016
(51) Int. Cl.: G01N 33/49, G01N 33/50, G01N 33/84

(54) **BLOOD TEST METHOD**
BLUT TEST METHODE
PROCEDE POUR TEST SANGUIN

(30) Priority: 06.03.2015 JP 2015045074; 25.11.2015 JP 2015230005
(43) Date of publication of application: 07.09.2016
(73) Proprietor: ARKRAY, Inc., Minami-ku Kyoto-shi, Kyoto 601-8045 (JP)
(72) Inventor: Nakamura, Tsutomu, Kyoto, 602-0008 (JP)
(74) Representative: Hoffmann Eitle

(56) References cited:
- CHRISTIANE ODDOZE ET AL: "Stability study of 81 analytes in human whole blood, in serum and in plasma", CLINICAL BIOCHEMISTRY, ELSEVIER INC, US, CA, vol. 45, no. 6, 6 January 2012 (2012-01-06), pages 464-469, XP028471930, ISSN: 0009-9120, DOI: 10.1016/J.CLINBIOCHEM.2012.01.012 [retrieved on 2012-01-18]
- MOHRI M ET AL: "Effects of heparin, citrate, and EDTA on plasma biochemistry of sheep: Comparison with serum", RESEARCH IN VETERINARY SCIENCE, BRITISH VETERINARY ASSOCIATION, LONDON, GB, vol. 86, no. 1, 1 February 2009 (2009-02-01), pages 111-114, XP025814688, ISSN: 0034-5288, DOI: 10.1016/J.RVSC.2008.05.010 [retrieved on 2008-06-24]

## Description

### BACKGROUND

### Technical Field

The present invention relates to a blood test method which is configured to measure a blood count and an electrolyte in a blood sample.

### Related Art

When a blood test is carried out, an anticoagulant is used for inhibiting coagulation of a blood sample which is to be used as a specimen. Examples of the anticoagulant include citric acid, sodium citrate, ethylenediaminetetraacetic acid (EDTA), and heparin. Usually, among these anticoagulants, different anticoagulants are used for different purposes (see, for example, Patent Document 1 and Non-patent Document 1).

For example, there is a report showing that heparin has a property that easily allows aggregation of white blood cells, and that the white blood cell aggregation increases with time. There is also a report showing that aggregation of platelets is found from immediately after blood collection in some specimens (see, for example, Non-patent Documents 2 and 3). Thus, it has been thought that accurate measurement of white blood cells and platelets is impossible in blood count tests using heparin.

When electrolyte measurement is carried out as a blood test, the measurement is impossible in cases where the anticoagulant contains Na or K. Thus, an anticoagulant which is free from Na and K, for example, heparin, is used.
Patent Document 1: Japanese National-Phase Publication (JP-A) No. 2008-510578
Non-patent Document 1: EDTA ... Selection of Anticoagulant in Blood Test. From Coulter No. 18 Sept, 1992
Non-patent Document 2: Shiro Miwa et al.: Influence of Various Anticoagulants on Blood Count. Rinsho Byori 15(5) 376-380, 1967
Non-patent Document 3: Akemi Shimasaki et al.: Platelet Aggregation Due to Mixing in Blood Collection Using Heparin. The Japanese Journal of Clinical Hematology 38(4) 323-330, 1997

Oddoze et al., discloses blood measurements employing K3-EDTA tubes for hematology analysis, and several other tubes for biochemistry analysis.

### SUMMARY OF THE INVENTION

### PROBLEM TO BE SOLVED BY THE INVENTION

In cases where different anticoagulants are used for different purposes, for example, heparin may be used for electrolyte measurement, and EDTA-2Na, 2K, and 3K may be used for blood count measurement. In such cases, a plurality of blood collection tubes containing different anticoagulants are necessary for measurement of a blood count and an electrolyte. As a result, blood collection becomes laborious, leading to an increase in the examination cost, and the burden on the patient from whom blood is collected increases.

Thus, a blood test method that is configured to measure both a blood count and an electrolyte in a single blood collection tube is preferred.

One embodiment of the invention aims to provide a blood test method which is configured to measure a blood count and an electrolyte in a blood sample.

### MEANS FOR SOLVING THE PROBLEMS

Specific means for achieving the object described above are as follows.
<1> One embodiment of the invention is a blood test method comprising: a providing step of providing a blood sample containing an anticoagulant containing a salt of ethylenediaminetetraacetic acid (EDTA) other than sodium salt, potassium salt, or calcium salt of EDTA; and a measurement step of measuring a blood count and an electrolyte in the blood sample.
<2> The blood test method according to <1>, wherein the measurement step measures a blood count and an electrolyte in the blood sample, and in addition, measures at least one item selected from the group consisting of a C-reactive protein (CRP), a serum amyloid protein A (SAA), a tumor necrosis factor (TNF), interleukin-1 (IL-1), α1-antitrypsin, α1-antichymotrypsin, α1-acidic glycoprotein, haptoglobin, ceruloplasmin, troponin I, CK-MB, myoglobin, and a heart-type fatty acid-binding protein (H-FABP) in the blood sample.
<3> The blood test method according to <1> or <2>, wherein the measurement step measures a blood count and an electrolyte in the blood sample, and in addition, measures a C-reactive protein (CRP) in the blood sample.
<4> One embodiment of the invention is the blood test method according to any one of <1> to <3>, wherein the EDTA salt is at least one salt selected from the group consisting of lithium salt, barium salt, cobalt salt, nickel salt, and ammonium salt of EDTA.
<5> One embodiment of the invention is the blood test method according to any one of <1> to <4>, wherein the EDTA salt is lithium salt of EDTA.
<6> One embodiment of the invention is the blood test method according to any one of <1> to <5>, wherein the EDTA salt is obtained by mixing EDTA with a metal hydroxide(s) (excluding hydroxides of sodium, potassium, or calcium) at a mass ratio of EDTA:metal hydroxide(s) of from 1: 0.1 to 1:0.6.
<7> One embodiment of the invention is the blood test method according to any one of <1> to <6>, wherein an EDTA salt concentration in the blood sample is from 0.5 mg/mL to 3.0 mg/mL.
<8> One embodiment of the invention is the blood test method according to any one of <1> to <7>, wherein an EDTA salt concentration in the blood sample is from 0.75 mg/mL to 1.5 mg/mL.

In addition, it is described:
(i) A blood test device comprising: a sample placement section in which a blood sample containing an anticoagulant containing a salt of ethylenediaminetetraacetic acid (EDTA) other than sodium salt, potassium salt, or calcium salt of EDTA is placed; and a measurement section by which measurement of a blood count and an electrolyte is carried out for the blood sample placed in the sample placement section.
(ii) The blood test device according to (i), wherein the measurement section has a blood counter configured to measure the blood count in the blood sample and an electrolyte measuring instrument configured to measure the electrolyte in the blood sample.
(iii) The blood test device according to (i) or (ii), wherein the measurement section measures a blood count and an electrolyte in the blood sample, and in addition, measures at least one item selected from the group consisting of a C-reactive protein (CRP), a serum amyloid protein A (SAA), a tumor necrosis factor (TNF), interleukin-1 (IL-1), α1-antitrypsin, α1-antichymotrypsin, α1-acidic glycoprotein, haptoglobin, ceruloplasmin, troponin I, CK-MB, myoglobin, and a heart-type fatty acid-binding protein (H-FABP) in the blood sample.
(iv) The blood test device according to any one of (i) to (iii), wherein the measurement section measures the blood count and the electrolyte in the blood sample, and in addition, measures a C-reactive protein (CRP) in the blood sample.

### EFFECT OF THE INVENTION

According to an embodiment of the invention, a blood test method which is configured to measure a blood count and an electrolyte in a blood sample can be provided.

### BRIEF DESCRIPTION OF THE DRAWINGS

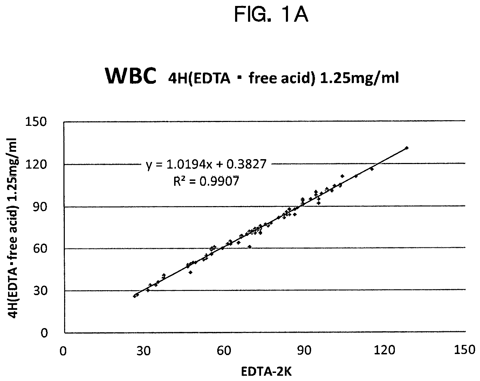

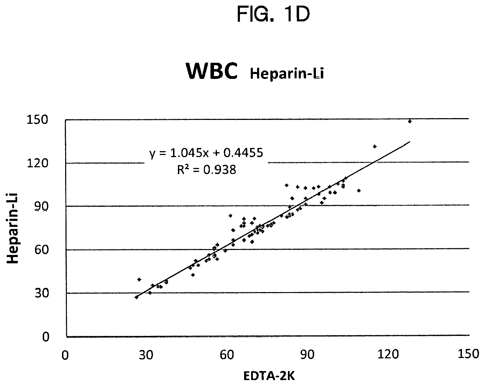

Fig. 1 (a) to (c) are graphs for comparison of results of counting of white blood cells between Blood Collection Tubes 1 to 3 (EDTA-4H + LiOH) and Blood Collection Tube 4 (EDTA-2K). Fig. 1 (d) is a graph for comparison of results of counting of white blood cells between Blood Collection Tube 5 (heparin Li) and Blood Collection Tube 4 (EDTA-2K).

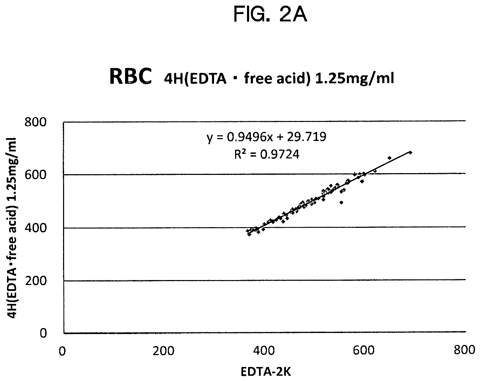

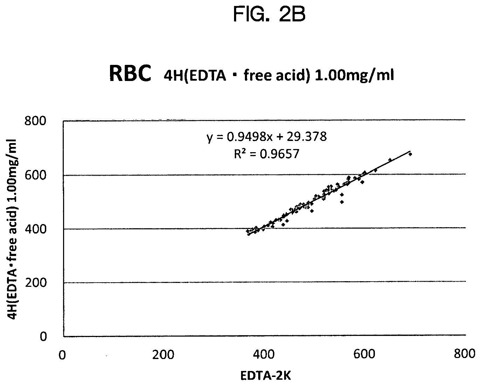

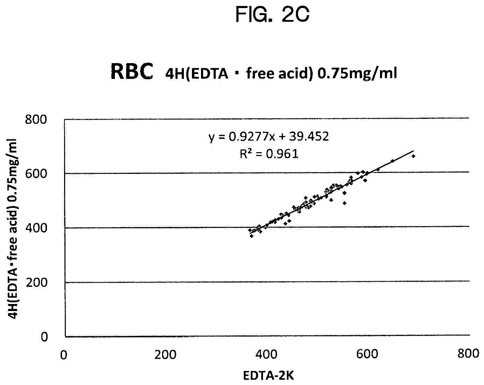

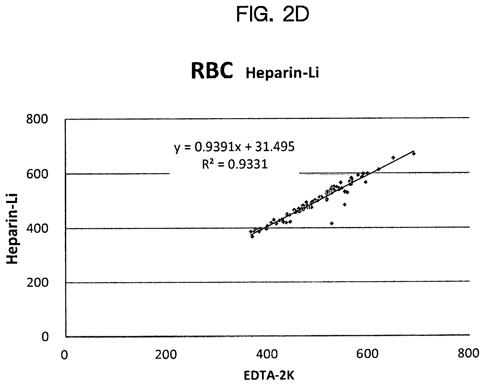

Fig. 2 (a) to (c) are graphs for comparison of results of counting of red blood cells between Blood Collection Tubes 1 to 3 (EDTA-4H + LiOH) and Blood Collection Tube 4 (EDTA-2K). Fig. 2 (d) is a graph for comparison of results of counting of red blood cells between Blood Collection Tube 5 (heparin Li) and Blood Collection Tube 4 (EDTA-2K).

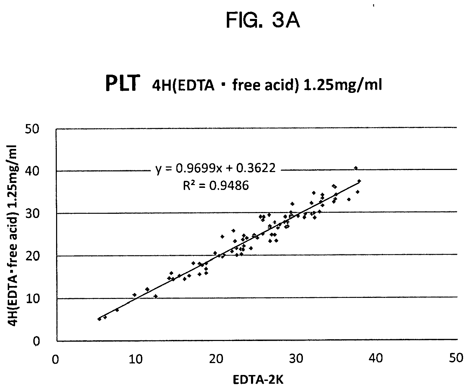

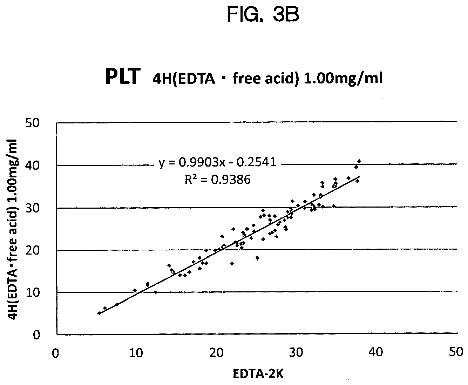

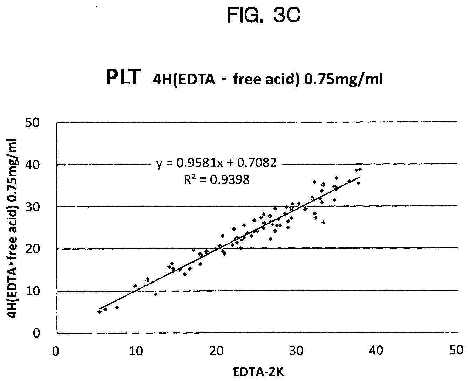

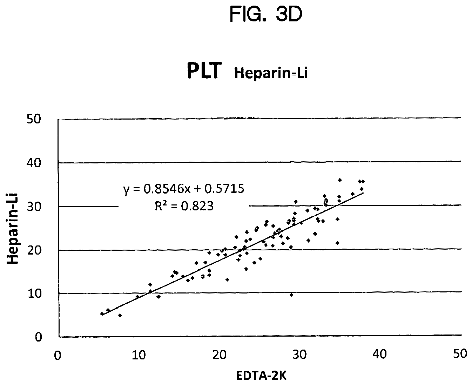

Fig. 3 (a) to (c) are graphs for comparison of results of counting of platelets between Blood Collection Tubes 1 to 3 (EDTA-4H + LiOH) and Blood Collection Tube 4 (EDTA-2K). Fig. 3 (d) is a graph for comparison of results of counting of platelets between Blood Collection Tube 5 (heparin Li) and Blood Collection Tube 4 (EDTA-2K).

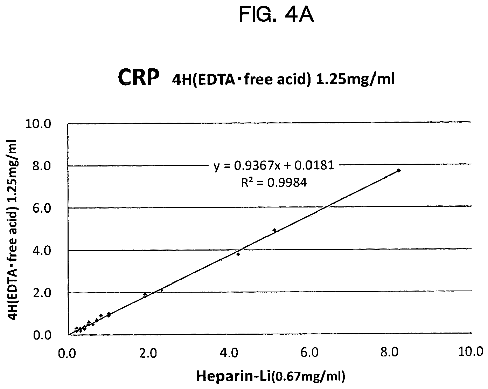

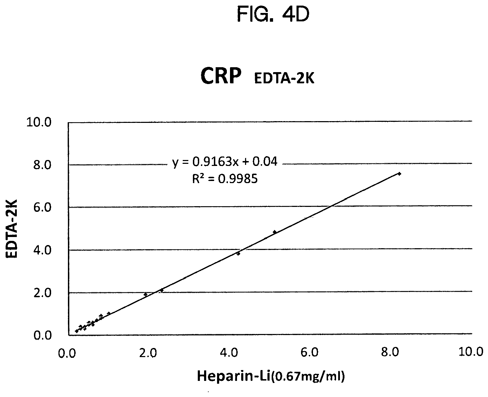

Fig. 4 (a) to (c) are graphs for comparison of results of CRP measurement between Blood Collection Tubes 1 to 3 (EDTA-4H + LiOH) and Blood Collection Tube 5 (heparin Li). Fig. 4 (d) is a graph for comparison of results of CRP measurement between Blood Collection Tube 4 (EDTA-2K) and Blood Collection Tube 5 (heparin Li).

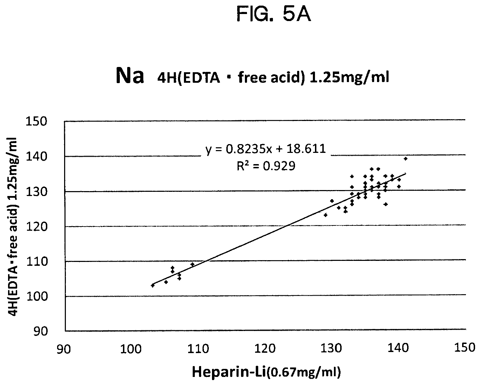

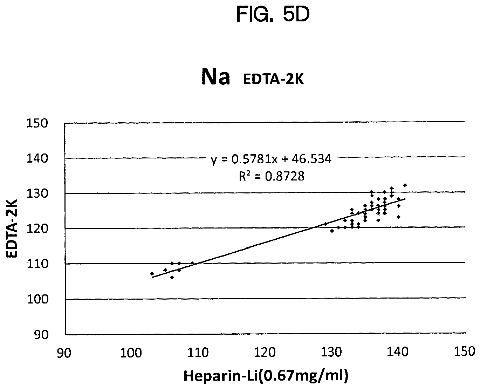

Fig. 5 (a) to (c) are graphs for comparison of results of measurement of the Na concentration between Blood Collection Tubes 1 to 3 (EDTA-4H + LiOH) and Blood Collection Tube 5 (heparin Li). Fig. 5 (d) is a graph for comparison of results of measurement of the Na concentration between Blood Collection Tube 4 (EDTA-2K) and Blood Collection Tube 5 (heparin Li).

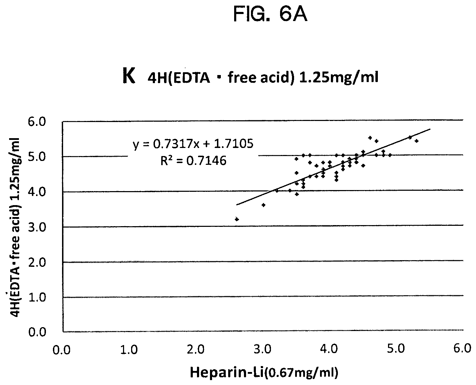

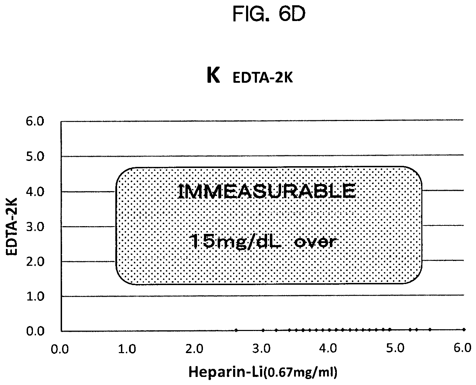

Fig. 6 (a) to (c) are graphs for comparison of results of measurement of the K concentration between Blood Collection Tubes 1 to 3 (EDTA-4H + LiOH) and Blood Collection Tube 5 (heparin Li). Fig. 6 (d) is a graph for comparison of results of measurement of the K concentration between Blood Collection Tube 4 (EDTA-2K) and Blood Collection Tube 5 (heparin Li).

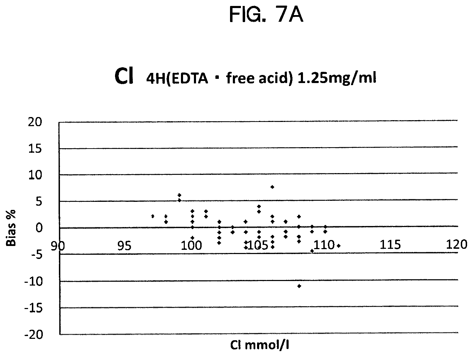

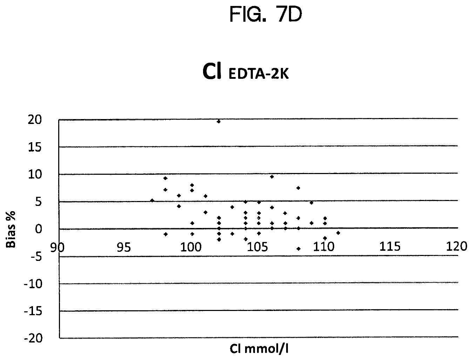

Fig. 7 (a) to (c) are graphs for comparison of results of measurement of the Cl concentration between Blood Collection Tubes 1 to 3 (EDTA-4H + LiOH) and Blood Collection Tube 5 (heparin Li). Fig. 7 (d) is a graph for comparison of results of measurement of the Cl concentration between Blood Collection Tube 4 (EDTA-2K) and Blood Collection Tube 5 (heparin Li).

### DETAILED DESCRIPTION OF THE INVENTION

One embodiment of the blood test method of the invention is described below. In addition, one embodiment of the blood test device for carrying out the blood test method is described.

In the present description, each numerical range expressed using "from ... to ..." means the range including the values described before and after "to" as the lower limit and the upper limit, respectively.

### [Blood Test Device]

The blood test device comprises a sample placement section in which a blood sample containing an anticoagulant containing a salt of ethylenediaminetetraacetic acid (EDTA) other than sodium salt, potassium salt, or calcium salt of EDTA is placed; and a measurement section by which measurement of a blood count and an electrolyte is carried out for the blood sample placed in the sample placement section.

Conventionally, different anticoagulants have been used for different purposes. Heparin has been used for measurement of an electrolyte, and EDTA-2Na, 2K, or 3K has been used for blood count measurement. Thus, in cases where a blood count and an electrolyte are to be measured, a plurality of blood collection tubes (blood sample containers) containing different anticoagulants are necessary. As a result, blood collection becomes laborious, leading to an increase in the examination cost, and the burden on the patient from whom blood is collected increases.

In the blood test device, a blood sample containing an anticoagulant containing a salt of EDTA other than sodium salt, potassium salt, or calcium salt of EDTA is placed in the sample placement section, and a blood count and an electrolyte in the blood sample placed in the sample placement section are measured by the measurement section. Therefore, use of different anticoagulants for different purposes is not necessary, and measurement of a blood count and an electrolyte in the blood sample can be carried out using a single type of anticoagulant or a single blood collection tube.

The blood sample to be subjected to the measurement using the blood test device is the same as the blood sample to be used in the blood test method of the present embodiment described later.

The blood test device comprises a sample placement section in which a blood sample containing an anticoagulant containing a salt of EDTA other than sodium salt, potassium salt, or calcium salt of EDTA is placed. The sample placement section is not limited as long as the sample placement section has a constitution in which a blood sample containing the anticoagulant containing the EDTA salt can be placed, and a blood count and an electrolyte in the blood sample can be measured by the measurement section described later. Examples of the sample placement section include a place where a blood collection tube (blood sample container) which contains a blood sample containing the anticoagulant containing the EDTA salt can be placed, and a container into which the blood sample can be injected.

The blood test device comprises a measurement section by which measurement of a blood count and an electrolyte is carried out for the blood sample placed in the sample placement section. The measurement section may comprise, for example, a nozzle for suction of the blood sample, and a measuring instrument to which the blood sample sucked by the nozzle is supplied for measurement of items.

In the measuring instrument, a device which can measure a blood count (a blood counter) and a device which can measure an electrolyte (an electrolyte measurement device) may be separately present, and the combination of these devices may constitute the system. Alternatively, the measuring instrument may be a single measuring device which can measure both a blood count and an electrolyte.

In cases where a blood count and an electrolyte in the blood sample are to be measured, these items may be measured at the same time, or may be sequentially measured.

The blood test device may be configured to measure the biochemical test items described later, in addition to a blood count and an electrolyte. For example, in the measuring instrument, a device which can measure a blood count (a blood counter), a device which can measure an electrolyte (an electrolyte measurement device), and a device which can measure a C-reactive protein (CRP) (CRP measurement device) may be separately present, and the combination of these devices may constitute the system. Alternatively, the measuring instrument may be a single measuring device which can measure a blood count, an electrolyte, and CRP.

### [Blood Test Method]

One embodiment of the blood test method of the invention comprises a providing step of providing a blood sample containing an anticoagulant containing a salt of ethylenediaminetetraacetic acid (EDTA) other than sodium salt, potassium salt, or calcium salt of EDTA; and a measurement step of measuring a blood count and an electrolyte in the blood sample.

The blood test method of the present embodiment may also be carried out using the blood test device described above.

By the blood test method of the present embodiment, a blood count and an electrolyte can be measured using a single blood collection tube. Therefore, the labor of blood collection and the examination cost can be reduced relative to cases where a blood count and an electrolyte are measured using a plurality of blood collection tubes containing different anticoagulants. Moreover, the burden on the patient from whom blood is collected can be reduced.

One embodiment of the blood test method of the invention uses an anticoagulant containing a salt of ethylenediaminetetraacetic acid (EDTA) other than sodium salt, potassium salt, or calcium salt of EDTA.

EDTA inhibits blood coagulation by formation of a soluble complex by chelation of calcium ions. Since EDTA salts are easily dissolved in solvents such as water, the anticoagulant to be used in the blood test method may be a solution in which an EDTA salt is dissolved in a solvent such as water. The anticoagulant to be used in the blood test method may also be a solid obtained by preparing a solution by dissolving an EDTA salt in a solvent such as water, and then drying the prepared solution.

The EDTA salt contained in the anticoagulant is not limited as long as the EDTA salt is an EDTA salt other than sodium salt, potassium salt, or calcium salt of EDTA. For example, the EDTA salt is preferably at least one salt selected from the group consisting of lithium salt, barium salt, cobalt salt, nickel salt, and ammonium salt of EDTA. In particular, the EDTA salt contained in the anticoagulant is preferably lithium salt of EDTA.

The EDTA salt contained in the anticoagulant is preferably a metal salt obtained by mixing EDTA with a metal hydroxide (excluding hydroxides of sodium, potassium, or calcium). The EDTA salt is more preferably a metal salt obtained by mixing EDTA with at least one metal hydroxide selected from the group consisting of lithium hydroxide, barium hydroxide, cobalt hydroxide, and nickel hydroxide (more preferably lithium hydroxide).

The EDTA salt contained in the anticoagulant is preferably obtained by mixing EDTA with a metal hydroxide(s) (excluding hydroxides of sodium, potassium, or calcium) at a mass ratio of EDTA:metal hydroxide(s) of from 1: 0.1 to 1:0.6. The mass ratio of EDTA:metal hydroxide(s) is more preferably from 1:0.2 to 1:0.6, still more preferably from 1:0.3 to 1:0.5.

In one embodiment of the blood test method of the invention, a blood sample containing the anticoagulant is provided, and the anticoagulant provided is used for measuring a blood count and an electrolyte.

In the blood sample to be used for the measurement of a blood count and an electrolyte, the EDTA salt concentration is preferably from 0.5 mg/mL to 3.0 mg/mL, more preferably from 0.75 mg/mL to 1.5 mg/mL, still more preferably from 0.75 mg/mL to 1.25 mg/mL.

In cases where the EDTA salt concentration is not less than 0.5 mg/mL, blood coagulation can be favorably inhibited. In cases where the EDTA salt concentration is not more than 3.0 mg/mL, hemolysis can be favorably suppressed.

In cases where the EDTA salt concentration is not less than 0.75 mg/mL, blood coagulation can be more favorably inhibited. In cases where the EDTA salt concentration is not more than 1.5 mg/mL (preferably not more than 1.25 mg/mL), hemolysis can be more favorably suppressed, and the influence of the EDTA salt on the measurement of electrolytes such as Na, K, and Cl can be reduced, enabling more accurate measurement. In particular, in cases where the EDTA salt is lithium salt, the influence of the EDTA salt on the measurement of electrolytes such as Na, K, and Cl can be more favorably reduced.

International Committee for Standardization in Hematology (ICSH) recommends inclusion of from 1.5 mg to 2.2 mg of EDTA salt per 1 mL of a blood sample (which corresponds to an EDTA salt concentration of from 1.5 mg/mL to 2.2 mg/mL) from the viewpoint of prevention of coagulation. The paragraph [0018] of JP-A No. 2008-510578 also describes that an EDTA concentration corresponding to about 1.5 mg of EDTA per 1 mL of blood is effective for prevention of coagulation.

In one embodiment of the invention, the concentration of the EDTA salt (preferably lithium salt of EDTA) may be from 0.75 mg/mL to 1.5 mg/mL, and, in such a case, blood coagulation can be suppressed using a smaller amount of EDTA relative to conventional cases. Furthermore, the influence of the EDTA salt on the measurement of electrolytes such as Na, K, and Cl can be reduced, enabling more accurate measurement.

The blood count to be measured by one embodiment of the blood test method of the invention provides information on cellular components of blood. For example, by the measurement of a blood count, white blood cells (WBC), red blood cells (RBC), hematocrit value (HCT), hemoglobin level (HGB), platelets (PLT), mean corpuscular volume (MCV), mean corpuscular hemoglobin (MCH), mean corpuscular hemoglobin concentration (MCHC), red cell distribution width (RDW), platelet distribution width (PDW), mean platelet volume (MPV), neutrophil count (NEUT), lymphocyte count (LYMPH), monocyte count (MONO), eosinophil count (EO), basophil count (BASO), reticulocyte count (RET), and reticulocyte ratio (RET%).

By the measurement of a blood count, at least one of white blood cells (WBC), red blood cells (RBC), or platelets (PLT) is preferably measured. More preferably, white blood cells (WBC), red blood cells (RBC), and platelets (PLT) are measured.

Examples of items which may be measured by one embodiment of the blood test method of the invention include, in addition to the blood count described above, electrolytes such as sodium (Na), potassium (K), and chlorine (CI).

Biochemical test items other than a blood count and an electrolyte may be measured (tested) by one embodiment of the blood test method of the invention. For example, in addition to a blood count and an electrolyte, the so-called blood components may be qualitatively or quantitatively tested by biochemical methods. Examples of the biochemical methods include a nitrogen content test, metal test, plasma protein-related test, colloid reaction, biochrome test, enzyme-related test, lipid test, and saccharometabolism test.

In one embodiment of the blood test method of the invention, at least one item selected from the group consisting of, for example, a C-reactive protein (CRP), a serum amyloid protein A (SAA), a tumor necrosis factor (TNF), interleukin-1 (IL-1), α1-antitrypsin, α1-antichymotrypsin, α1-acidic glycoprotein, haptoglobin, ceruloplasmin, troponin I, CK-MB, myoglobin, and heart-type a fatty acid-binding protein (H-FABP) in the blood sample may be additionally measured.

In one embodiment of the blood test method of the invention, the C-reactive protein (CRP) in the blood sample is preferably measured in addition to the blood count and the electrolyte in the blood sample. By such measurement, all of the blood count, CRP, and the electrolyte in the blood sample can be measured using a single type of anticoagulant or a single blood collection tube.

Examples of other items that may be measured in the test include general biochemical test items such as creatine phosphokinase (CPK), blood urea nitrogen (BUN), lactate dehydrogenase (LDH), alkaline phosphatase (ALP), GOT (glutamic oxaloacetic transaminase), GPT (glutamic pyruvic transaminase), triglyceride (TG), urea (UA), total cholesterol (TCHO), high-density lipoprotein cholesterol (HDLC), total protein (TP), albumin (ALB), amylase (AMYL), γ-glutamine transpeptidase (GGT), creatinine (CREA), and total bilirubin (TBIL). One or more of these items may be measured.

### EXAMPLES

One embodiment of the invention is described below by way of Examples. However, the invention is not limited to the Examples.

### <Experiment 1-Observation of Anticoagulant Property>

The anticoagulant property of the anticoagulant to be used in an embodiment of the blood test method of the invention was observed as follows.

### [Preparation of Aqueous Solutions 1 to 3]

Three kinds of aqueous solutions containing EDTA-4H (manufactured by Dojindo Laboratories) and LiOH (manufactured by Wako Pure Chemical Industries, Ltd.) (anticoagulants) were prepared as follows.
<Aqueous Solution 1> 30 mg of EDTA-4H and 11.9 mg of LiOH were dissolved in 1 mL of distilled water to prepare Aqueous Solution 1.
<Aqueous Solution 2> 20 mg of EDTA-4H and 7.9 mg of LiOH were dissolved in 1 mL of distilled water to prepare Aqueous Solution 2.
<Aqueous Solution 3> 10 mg of EDTA-4H and 4.0 mg of LiOH were dissolved in 1 mL of distilled water to prepare Aqueous Solution 3.

### [Samples]

As specimens (Sample), whole blood collected from subjects was used.

The number of specimens was 43, and the specimens included 31 specimens from males (Male) and 12 specimens from females (Female).

### [Experimental Method]

In a plastic container, 50 µL of each of the thus prepared Aqueous Solutions 1 to 3 was placed, and then dried. Subsequently, 1 mL of whole blood was added to each plastic container to provide samples with EDTA concentrations (Conc) in the whole blood of 1.5 mg/mL, 1.0 mg/mL, and 0.5 mg/mL, respectively. After the addition of the whole blood to each container, each sample was left to stand at room temperature for 1.5, 3.0, or 4.5 hours (90, 180, or 270 minutes), followed by observation of whether coagulation (Coagulation) and hemolysis (Hemolysis) occurred. More specifically, the state of coagulation was visually observed at Hour 1.5, 3.0, and 4.5. In addition, the specimens at Hour 1.5, 3.0, and 4.5 were centrifuged, and the state of hemolysis was visually observed after the centrifugation.

The results are shown in Table 1.

**[Table 1]**

| Conc mg/ml | 0.5 | | 1.0 | | 1.5 | |
|---|---|---|---|---|---|---|
| Time (min) | 90 | | 180 | | 270 | |
| Test | Coagulation | Hemolysis | Coagulation | Hemolysis | Coagulation | Hemolysis |
| Sample | 2/43 | 0/41 | 0/43 | 0/43 | 0/43 | 3/43 |
| Male | 0/31 | 0/31 | 0/31 | 0/31 | 0/31 | 3/31 |
| Female | 2/12 | 0/10 | 0/12 | 0/12 | 0/12 | 0/12 |

As shown in Table 1, in the cases where the EDTA concentration in the whole blood was 0.5 mg/mL or 1.5 mg/mL, most specimens showed no coagulation or hemolysis. In the cases where the EDTA concentration in the whole blood was 1.0 mg/mL, no specimen showed coagulation or hemolysis.

### <Experiment 2-Influence on Measured Values of Na, K, and Cl>

The influence of the anticoagulant to be used in one embodiment of the blood test method of the invention on the measured values of Na, K, and CI was studied.

### [Preparation of Aqueous Solutions 4 to 11]

Two kinds of aqueous solutions containing EDTA-4H (manufactured by Dojindo Laboratories) and LiOH (manufactured by Wako Pure Chemical Industries, Ltd.) (anticoagulants) were prepared as follows.
<Aqueous Solutions 4 to 7> In 1 mL of distilled water, 60 mg of EDTA-4H and 23.7 mg of LiOH were dissolved to prepare Aqueous Solution 4. Aqueous Solution 4 was diluted at a ratio of 2/3, 1/2, or 1/3 to prepare aqueous solutions. Thus, 4 kinds of aqueous solutions 4 to 7 having different concentrations were provided.
<Aqueous Solutions 8 to 11> In 1 mL of distilled water, 48 mg of EDTA-2NH₄ was dissolved to prepare Aqueous Solution 8. Aqueous Solution 8 was diluted at a ratio of 2/3, 1/2, or 1/3 to prepare aqueous solutions. Thus, 4 kinds of aqueous solutions 8 to 11 having different concentrations were provided.

### [Specimens]

As a specimen, a commercially available control serum was used.

To 0.57 mL of the commercially available control serum, 0.03 mL of each of Aqueous Solutions 4 to 11 was added to prepare measurement samples. In addition, distilled water was added to the commercially available control serum to prepare comparative measurement samples.

### [Experimental Method]

Using the thus prepared measurement samples, the concentration of each of Na, K, and Cl was measured five times, and significance test (significance level, 5%; significance was assumed at t≥2.2622) was carried out. The measured values were compared against the values obtained with the comparative measurement samples, in which distilled water was added.

The results are shown in Tables 2 to 4. Table 2 corresponds to the concentration of Na; Table 3 corresponds to the concentration of K; and Table 4 corresponds to the concentration of Cl.

**[Table 2]**

| | Distilled water | Aqueous solution 4 EDTA-4H 3.0 mg/mL +LiOH | Aqueous solution 5 EDTA-4H 2.0 mg/mL +LiOH | Aqueous solution 6 EDTA-4H 1.5 mg/mL +LiOH | Aqueous solution 7 EDTA-4H 1.0 mg/mL +LiOH | Aqueous solution 8 EDTA-2NH₄ 2.4 mg/mL | Aqueous solution 9 EDTA-2NH₄ 1.6 mg/mL | Aqueous solution 10 EDTA-2NH₄ 1.2 mg/mL | Aqueous solution 11 EDTA-2NH₄ 0.8 mg/mL |
|---|---|---|---|---|---|---|---|---|---|
| 1 | 129 | 126 | 128 | 129 | 129 | 124 | 128 | 129 | 130 |
| 2 | 129 | 125 | 127 | 130 | 131 | 125 | 127 | 129 | 130 |
| 3 | 131 | 126 | 124 | 129 | 138 | 126 | 128 | 130 | 131 |
| 4 | 131 | 127 | 127 | 129 | 130 | 125 | 128 | 130 | 131 |
| 5 | 132 | 124 | 127 | 130 | 130 | 125 | 128 | 130 | 130 |
| Mean | 130 | 126 | 127 | 129 | 132 | 125 | 128 | 130 | 130 |
| Standard deviation | 1.3 | 1.1 | 1.5 | 0.5 | 3.6 | 0.7 | 0.4 | 0.5 | 0.5 |
| Coefficient of variation (%) | 1.0 | 0.9 | 1.2 | 0.4 | 2.8 | 0.6 | 0.3 | 0.4 | 0.4 |
| p | - | 0.00 | 0.00 | 0.16 | 0.51 | 0.00 | 0.00 | 0.25 | 1.00 |
| t | - | 5.71 | 4.02 | 1.53 | 0.69 | 7.31 | 3.94 | 1.22 | 0.00 |

**[Table 3]**

| | Distilled water | Aqueous solution 4 EDTA-4H 3.0 mg/mL +LiOH | Aqueous solution 5 EDTA-4H 2.0 mg/mL +LiOH | Aqueous solution 6 EDTA-4H 1.5 mg/mL +LiOH | Aqueous solution 7 EDTA-4H 1.0 mg/mL +LiOH | Aqueous solution 8 EDTA-2NH₄ 2.4 mg/mL | Aqueous solution 9 EDTA-2NH₄ 1.6 mg/mL | Aqueous solution 10 EDTA-2NH₄ 1.2 mg/mL | Aqueous solution 11 EDTA-2NH₄ 0.8 mg/mL |
|---|---|---|---|---|---|---|---|---|---|
| 1 | 4.6 | 4.4 | 4.3 | 4.3 | 4.5 | 4.6 | 4.5 | 4.5 | 4.5 |
| 2 | 4.4 | 4.4 | 4.4 | 4.5 | 4.6 | 4.6 | 4.6 | 4.5 | 4.0 |
| 3 | 4.6 | 4.4 | 4.2 | 4.5 | 4.7 | 4.5 | 4.6 | 4.6 | 4.5 |
| 4 | 4.8 | 4.4 | 4.6 | 4.6 | 4.7 | 4.5 | 4.6 | 4.6 | 4.6 |
| 5 | 4.8 | 4.3 | 4.3 | 4.6 | 4.5 | 4.6 | 4.6 | 4.6 | 4.5 |
| Mean | 4.6 | 4.4 | 4.4 | 4.5 | 4.6 | 4.6 | 4.6 | 4.6 | 4.4 |
| Standard deviation | 0.1 | 0.0 | 0.2 | 0.1 | 0.1 | 0.1 | 0.0 | 0.1 | 0.2 |
| Coefficient of variation (%) | 3.1 | 1.0 | 3.5 | 2.7 | 2.2 | 1.2 | 1.0 | 1.2 | 5.4 |
| p | - | 0.01 | 0.03 | 0.27 | 1.00 | 0.57 | 0.77 | 0.57 | 0.18 |
| t | - | 3.21 | 2.53 | 1.19 | 0.00 | 0.59 | 0.30 | 0.59 | 1.44 |

**[Table 4]**

| | Distilled water | Aqueous solution 4 EDTA-4H 3.0 mg/mL +LiOH | Aqueous solution 5 EDTA-4H 2.0 mg/mL +LiOH | Aqueous solution 6 EDTA-4H 1.5 mg/mL +LiOH | Aqueous solution 7 EDTA-4H 1.0 mg/mL +LiOH | Aqueous solution 8 EDTA-2NH₄ 2.4 mg/mL | Aqueous solution 9 EDTA-2NH₄ 1.6 mg/mL | Aqueous solution 10 EDTA-2NH₄ 1.2 mg/mL | Aqueous solution 11 EDTA-2NH₄ 0.8 mg/mL |
|---|---|---|---|---|---|---|---|---|---|
| 1 | 94 | 91 | 93 | 94 | 92 | 96 | 92 | 94 | 94 |
| 2 | 92 | 94 | 94 | 92 | 93 | 93 | 94 | 93 | 94 |
| 3 | 93 | 95 | 94 | 94 | 91 | 95 | 95 | 95 | 94 |
| 4 | 90 | 92 | 96 | 92 | 95 | 96 | 96 | 94 | 94 |
| 5 | 92 | 95 | 94 | 94 | 95 | 96 | 97 | 95 | 93 |
| Mean | 92 | 93 | 94 | 93 | 93 | 95 | 95 | 94 | 94 |
| Standard deviation | 1.5 | 1.8 | 1.1 | 1.1 | 1.8 | 1.3 | 1.9 | 0.8 | 0.4 |
| Coefficient of variation (%) | 1.6 | 1.9 | 1.2 | 1.2 | 1.9 | 1.4 | 2.0 | 0.9 | 0.5 |
| p | - | 0.29 | 0.04 | 0.26 | 0.36 | 0.01 | 0.04 | 0.03 | 0.05 |
| t | - | 1.14 | 2.38 | 1.20 | 0.96 | 3.29 | 2.35 | 2.57 | 2.27 |

From Tables 2 to 4, it can be assumed that the measured values of Na, K, and Cl are least influenced by use of Aqueous solution 7, which contains LiOH and 1.0 mg/mL EDTA-4H.

### <Experiment 3-Measurement of Blood Count, CRP, and Electrolyte>

By one embodiment of the blood test method of the invention, a blood count (white blood cell count, red blood cell count, and platelet count), CRP, and an electrolyte in blood samples (whole blood) were measured.

### [Providing Blood Collection Tubes 1 to 3]

Blood Collection Tubes 1 to 3, which contain EDTA-4H (manufactured by Dojindo Laboratories) and LiOH (manufactured by Wako Pure Chemical Industries, Ltd.), were provided as follows.
<Blood Collection Tube 1> In distilled water, 300 mg of EDTA-4H and 118.5 mg of LiOH were dissolved such that 5 mL of an aqueous solution was prepared. In a blood collection tube, 10.4 µL of the prepared aqueous solution was placed, and then dried.
<Blood Collection Tube 2> In distilled water, 300 mg of EDTA-4H and 118.5 mg of LiOH were dissolved such that 5 mL of an aqueous solution was prepared. In a blood collection tube, 8.3 µL of the prepared aqueous solution was placed, and then dried.
<Blood Collection Tube 3> In distilled water, 300 mg of EDTA-4H and 118.5 mg of LiOH were dissolved such that 5 mL of an aqueous solution was prepared. In a blood collection tube, 6.3 µL of the prepared aqueous solution was placed, and then dried.

### [Experimental Method]

To each of the Blood Collection Tubes 1 to 3 described above, 0.5 mL of whole blood as a specimen was fed to provide samples with EDTA concentrations in the whole blood of 1.25 mg/mL, 1.0 mg/mL, and 0.75 mg/mL, respectively. Thereafter, a blood count, CRP, and an electrolyte were measured using the Blood Collection Tubes 1 to 3 to which the whole blood was fed. The measurement of a blood count, CRP, and an electrolyte was carried out using SB-1420 manufactured by Arkray, Inc. and D-Concept D-01 and D-02 manufactured by Arkray.

### [Samples for Comparison]

For comparison, Blood Collection Tube 4 containing EDTA-2K (product name, NIHON) and Blood Collection Tube 5 containing heparin Li (prepared by dissolving 300 mg of heparin Li in distilled water such that 5 mL of an aqueous solution was prepared, placing 8.3 µL of the prepared aqueous solution in a blood collection tube, and then drying the solution) were provided. A blood count was measured by Blood Collection Tube 4, and CRP and an electrolyte were measured using Blood Collection Tube 5.

### (Blood Count)

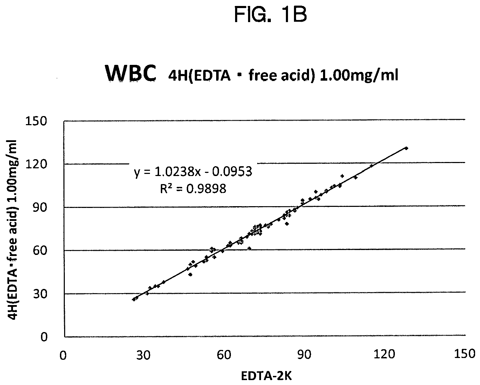

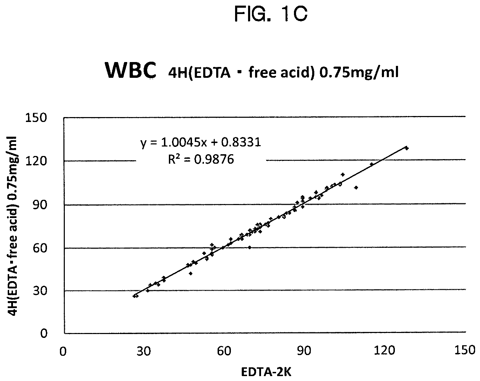

Figs. 1 to 3 show the results of measurement of the blood count (white blood cell count, red blood cell count, and platelet count). In each of Figs. 1 to 3, (a) to (c) show comparison of the results of a blood count between Blood Collection Tubes 1 to 3 (EDTA-4H + LiOH) and Blood Collection Tube 4 (EDTA-2K), and (d) shows comparison of the results of a blood count between Blood Collection Tube 5 (heparin Li) and Blood Collection Tube 4 (EDTA-2K).

Fig. 1 shows comparison of the results of measurement of the white blood cell (WBC) count; Fig. 2 shows comparison of the results of measurement of the red blood cell (RBC) count; and Fig. 3 shows comparison of the results of measurement of the platelet (PLT) count

As shown in Fig. 1 (a) to (c), wherein the white blood cell (WBC) count in each of Blood Collection Tubes 1 to 3 is taken along the ordinate, and the white blood cell (WBC) count in Blood Collection Tube 4 is taken along the abscissa, the slope of the regression equation was about 1 (satisfying the range of from 0.9 to 1.1), and the correlation coefficient (R²) was not less than 0.9.

As shown in Fig. 2 (a) to (c), wherein the red blood cell (RBC) count in each of Blood Collection Tubes 1 to 3 is taken along the ordinate, and the red blood cell (RBC) count in Blood Collection Tube 4 is taken along the abscissa, the slope of the regression equation was within the range of from 0.9 to 1.0, and the correlation coefficient (R²) was not less than 0.9.

As shown in Fig. 3 (a) to (c), wherein the platelet (PLT) count in each of Blood Collection Tubes 1 to 3 is taken along the ordinate, and the platelet (PLT) count in Blood Collection Tube 4 is taken along the abscissa, the slope of the regression equation was within the range of from 0.9 to 1.0, and the correlation coefficient (R²) was not less than 0.9.

Thus, by using the blood collection tubes containing EDTA-4H + LiOH, the performance of measurement of the blood count (white blood cell count, red blood cell count, and platelet count) could be secured.

As shown in Figs. 1 to 3, by using a blood collection tube containing EDTA-4H + LiOH, the blood count (white blood cell count, red blood cell count, and platelet count) could be more accurately measured relative to the cases where a blood collection tube containing heparin Li was used (Figs. 1 (d) to 3 (d)).

### (CRP)

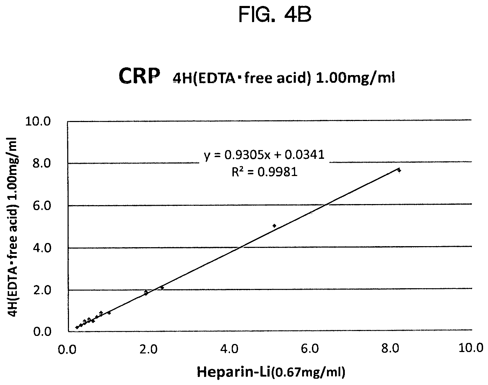

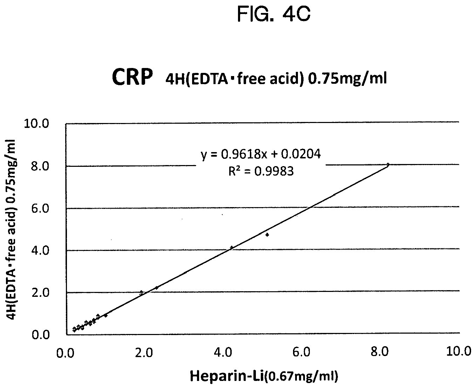

The results of measurement of CRP (a C-reactive protein) are shown in Fig. 4. Fig. 4 (a) to (c) show comparison of the results of CRP measurement between Blood Collection Tubes 1 to 3 (EDTA-4H + LiOH) and Blood Collection Tube 5 (heparin Li), and Fig. 4 (d) shows comparison of the results of CRP measurement between Blood Collection Tube 4 (EDTA-2K) and Blood Collection Tube 5 (heparin Li).

As shown in Fig. 4 (a) to (c), wherein the CRP measurement value in each of Blood Collection Tubes 1 to 3 is taken along the ordinate, and the CRP measurement value in Blood Collection Tube 5 is taken along the abscissa, the slope of the regression equation was from 0.9 to 1.0, and the correlation coefficient (R²) was not less than 0.9.

Thus, by using the blood collection tubes containing EDTA-4H + LiOH, the performance of measurement of CRP could be secured.

### (Electrolyte)

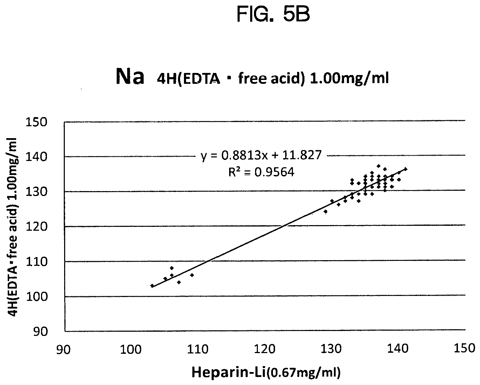

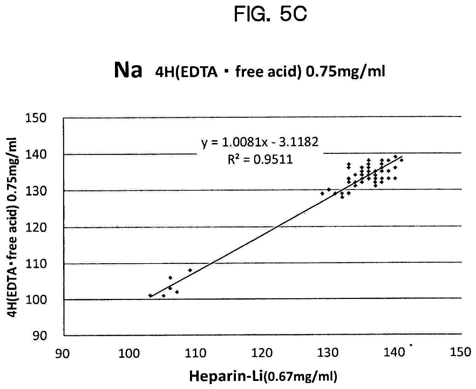

Fig. 5 to 7 show the results of measurement of an electrolyte (Na, K, and CI). In each of Figs. 5 to 7, (a) to (c) show comparison of the results of an electrolyte measurement between Blood Collection Tubes 1 to 3 (EDTA-4H + LiOH) and Blood Collection Tube 5 (heparin Li), and (d) shows comparison of the results of an electrolyte measurement between Blood Collection Tube 4 (EDTA-2K) and Blood Collection Tube 5 (heparin Li).

As shown in Fig. 6 (d), wherein Blood Collection Tube 4 (EDTA-2K) was used, measurement of potassium (K) was impossible (due to over-ranging).

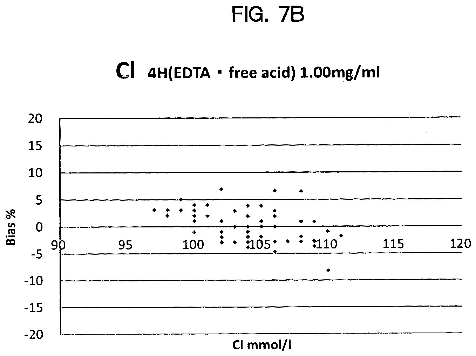

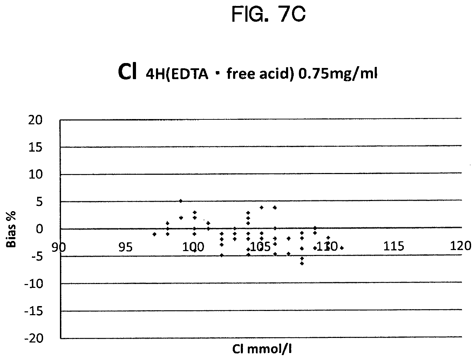

In Fig. 7, the measured value of chlorine (Cl) obtained using Blood Collection Tube 5 (heparin Li) was provided as a control, and judgment was made based on the difference from the control (Bias%).

As shown in Fig. 5 (a) to (c), wherein the measured concentration of sodium (Na) in each of Blood Collection Tubes 1 to 3 is taken along the ordinate, and the measured concentration of sodium (Na) in Blood Collection Tube 5 is taken along the abscissa, the slope of the regression equation was not less than 0.8, and the correlation coefficient (R²) was not less than 0.9.

On the other hand, as shown in Fig. 5 (d), wherein the measured concentration of sodium (Na) in Blood Collection Tube 4 is taken along the ordinate, and the measured concentration of sodium (Na) in Blood Collection Tube 5 is taken along the abscissa, the slope of the regression equation was about 0.58, and the correlation coefficient (R²) was less than 0.9.

Thus, as shown in Fig. 5, by using a blood collection tube containing EDTA-4H + LiOH, the sodium (Na) concentration could be more accurately measured relative to the cases where a blood collection tube containing EDTA-2K was used (Fig. 5 (d)).

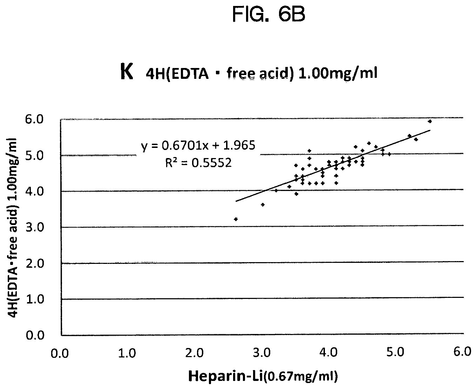

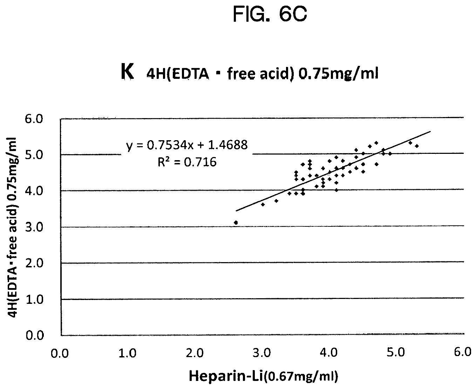

As shown in Fig. 6, measurement of the potassium (K) concentration was impossible in the cases where a blood collection tube containing EDTA-2K was used (Fig. 6 (d)). However, measurement of the potassium (K) concentration was possible in the cases where a blood collection tube containing EDTA-4H + LiOH was used.

As shown in Fig. 7, the difference in the values was mostly within the range of from -10 to +10% between the cases where a blood collection tube containing EDTA-4H+LiOH was used and the cases where a blood collection tube containing heparin Li was used. However, the difference in the values was large and exceeded +15% in some data between the cases where a blood collection tube containing EDTA-2K was used (Fig. 7 (d)) and the cases where a blood collection tube containing heparin Li was used.

Thus, as shown in Fig. 7, by using a blood collection tube containing EDTA-4H + LiOH, the chlorine (Cl) concentration could be more accurately measured relative to the cases where a blood collection tube containing EDTA-2K was used (Fig. 7 (d)).

Thus, by one embodiment of the blood test method of the invention, measurement of a blood count and an electrolyte in blood samples (whole blood) was possible, and measurement of CRP in blood samples was also possible. Accordingly, by the blood test method, measurement of a blood count (white blood cell count, red blood cell count, and platelet count), CRP, and an electrolyte can be carried out in a single blood collection tube, and the number of blood collection tubes can therefore be reduced. Thus, the labor and the cost of blood collection can be reduced, and the burden on patients can be reduced.

## Claims

1. A blood test method comprising:
providing a blood sample containing an anticoagulant containing ethylenediaminetetraacetic acid (EDTA) salt other than sodium salt, potassium salt, or calcium salt of EDTA; and
measuring a blood count and an electrolyte in the blood sample.

2. The blood test method according to claim 1, further comprising measuring at least one item selected from the group consisting of a C-reactive protein (CRP), a serum amyloid protein A (SAA), a tumor necrosis factor (TNF), interleukin-1 (IL-1), α1-antitrypsin, α1-antichymotrypsin, α1-acidic glycoprotein, haptoglobin, ceruloplasmin, troponin I, CK-MB, myoglobin, and a heart-type fatty acid-binding protein (H-FABP) in the blood sample.

3. The blood test method according to claim 1 or claim 2, wherein a C-reactive protein (CRP) is measured in the blood sample.

4. The blood test method according to any one of claim 1 to claim 3, wherein the EDTA salt is at least one salt selected from the group consisting of lithium salt, barium salt, cobalt salt, nickel salt, and ammonium salt of EDTA.

5. The blood test method according to any one of claim 1 to claim 4, wherein the EDTA salt is lithium salt of EDTA.

6. The blood test method according to any one of claim 1 to claim 5, wherein the EDTA salt is obtained by mixing EDTA with a metal hydroxide(s) (excluding hydroxides of sodium, potassium, or calcium) at a mass ratio of EDTA:metal hydroxide(s) of from 1: 0.1 to 1:0.6.

7. The blood test method according to any one of claim 1 to claim 6, wherein an EDTA salt concentration in the blood sample is from 0.5 mg/mL to 3.0 mg/mL.

8. The blood test method according to any one of claim 1 to claim 7, wherein an EDTA salt concentration in the blood sample is from 0.75 mg/mL to 1.5 mg/mL.

## Patentansprüche

1. Bluttestverfahren, umfassend:
Bereitstellen einer Blutprobe, enthaltend ein Antikoagulans, enthaltend Ethylendiamintetraessigsäure (EDTA)-Salz, das ein anderes als Natriumsalz, Kaliumsalz oder Calciumsalz von EDTA ist; und
Bestimmen eines Blutbilds und eines Elektrolyts in der Blutprobe.

2. Bluttestverfahren gemäß Anspruch 1, weiterhin umfassend Bestimmen wenigstens eines Parameters, ausgewählt aus der Gruppe, bestehend aus einem C-reaktiven Protein (CRP), einem Serum-Amyloidprotein A (SAA), einem Tumornekrosefaktor (TNF), Interleukin-1 (IL-1), α1-Antitrypsin, α1-Antichymotrypsin, α1-saurem Glykoprotein, Haptoglobin, Ceruloplasmin, Troponin I, CK-MB, Myoglobin und einem Herztyp-Fettsäure-bindenden Protein (H-FABP), in der Blutprobe.

3. Bluttestverfahren gemäß Anspruch 1 oder Anspruch 2, wobei ein C-reaktives Protein (CRP) in der Blutprobe bestimmt wird.

4. Bluttestverfahren gemäß einem der Ansprüche 1 bis 3, wobei das EDTA-Salz wenigstens ein Salz ist, ausgewählt aus der Gruppe, bestehend aus Lithiumsalz, Bariumsalz, Kobaltsalz, Nickelsalz und Ammoniumsalz von EDTA.

5. Bluttestverfahren gemäß einem der Ansprüche 1 bis 4, wobei das EDTA-Salz Lithiumsalz von EDTA ist.

6. Bluttestverfahren gemäß einem der Ansprüche 1 bis 5, wobei das EDTA-Salz durch Vermischen von EDTA mit Metallhydroxid(en) (mit Ausnahme von Hydroxiden von Natrium, Kalium oder Calcium) bei einem Massenverhältnis von EDTA:Metallhydroxid(en) von 1:0,1 bis 1:0,6 erhalten wird.

7. Bluttestverfahren gemäß einem der Ansprüche 1 bis 6, wobei die EDTA-Salzkonzentration in der Blutprobe von 0,5 mg/mL bis 3,0 mg/mL beträgt.

8. Bluttestverfahren gemäß einem der Ansprüche 1 bis 7, wobei die EDTA-Salzkonzentration in der Blutprobe von 0,75 mg/mL bis 1,5 mg/mL beträgt.

## Revendications

1. Procédé de test sanguin comprenant :
la mise à disposition d'un échantillon de sang contenant un anticoagulant contenant un sel d'acide éthylènediaminetétraacétique (EDTA) autre qu'un sel de sodium, sel de potassium, ou sel de calcium d'EDTA ; et
la mesure de la numération globulaire et d'un électrolyte dans l'échantillon de sang.

2. Procédé de test sanguin selon la revendication 1, comprenant en outre la mesure d'au moins un élément sélectionné dans le groupe consistant en la protéine C-réactive (CRP), la protéine amyloïde A sérique (SAA), le facteur de nécrose tumorale (TNF), l'interleukine-1 (IL-1), l'α1-antitrypsine, l'α1-antichymotrypsine, l'α1-glycoprotéine acide, l'haptoglobine, la céruloplasmine, la troponine I, la CK-MB, la myoglobine, et la protéine de liaison aux acides gras de type cardiaque (H-FABP) dans l'échantillon de sang.

3. Procédé de test sanguin selon la revendication 1 ou la revendication 2, dans lequel la protéine C-réactive (CRP) est mesurée dans l'échantillon de sang.

4. Procédé de test sanguin selon l'une quelconque de la revendication 1 à la revendication 3, dans lequel le sel d'EDTA est au moins un sel sélectionné dans le groupe consistant en un sel de lithium, un sel de baryum, un sel de cobalt, un sel de nickel, et un sel d'ammonium d'EDTA.

5. Procédé de test sanguin selon l'une quelconque de la revendication 1 à la revendication 4, dans lequel le sel d'EDTA est un sel de lithium d'EDTA.

6. Procédé de test sanguin selon l'une quelconque de la revendication 1 à la revendication 5, dans lequel le sel d'EDTA est obtenu en mélangeant de l'EDTA avec un/des hydroxyde(s) métallique(s) (à l'exclusion des hydroxydes de sodium, potassium, ou calcium) selon un rapport en masse d'EDTA:hydroxyde(s) métallique(s) compris entre 1:0,1 et 1:0,6.

7. Procédé de test sanguin selon l'une quelconque de la revendication 1 à la revendication 6, dans lequel la concentration en sel d'EDTA dans l'échantillon de sang est comprise entre 0,5 mg/ml et 3,0 mg/ml.

8. Procédé de test sanguin selon l'une quelconque de la revendication 1 à la revendication 7, dans lequel la concentration en sel d'EDTA dans l'échantillon de sang est comprise entre 0,75 mg/ml et 1,5 mg/ml.
